# EUROPEAN PATENT APPLICATION

(11) **EP 4 162 882 A1**
(43) Date of publication of application: **12.04.2023**
(21) Application number: 21201472.4
(22) Date of filing: 07.10.2021
(51) Int. Cl.: A61B 6/04, A61B 5/00, A61B 6/00, G06V 40/10

(54) **COMPUTER-IMPLEMENTED METHOD FOR POSITIONING A PATIENT FOR X-RAY IMAGE RECORDING**

(71) Applicant: AGFA NV, 2640 Mortsel (BE)
(72) Inventor: BEHIELS, Gert, 2640 Mortsel (BE)
(74) Representative: Verbrugghe, Anne Marie L.

(57) **Abstract**

Method for guiding a patient to a position suitable for image recording by showing an instruction video in which location and pose changes for moving from a recorded current position to an acceptable recording pose and position are shown.

## Description

### Technical Field

The present invention relates to a computer-implemented method for positioning a patient for x-ray image recording.

### Background of the invention

### Summary of invention

X-ray imaging is a non-invasive technique to capture medical images of patients or animals.

Image recording comprises irradiating an object with an X-ray beam. As the X-ray beam passes through the object, it is attenuated by a degree that varies with the internal composition and/or thickness of the object. An X-ray detector is then arranged to capture the image-wise modulated X-ray beam and to convert the image-wise modulated X-ray beam into an image showing the internal structure of the object.

In the field of radiographic image recording it is nowadays common to use re-usable radiography detectors (also called 'panels', 'flat panels', 'flat panel detectors' etc.) for X-ray image recording.

Different types of such re-usable detectors exist.

One type of X-ray recording devices used in so-called computed radiography (CR) comprises a photo-stimulable phosphor detector which stores image-wise modulated radiation when it is exposed to an X-ray image.

To release the stored image, the photostimulable phosphor detector is scanned by means of stimulating radiation, e.g. by means of laser light of an appropriate wavelength adapted to the type photostimulable phosphor.

Upon stimulation the phosphor detector releases the stored radiation image in the form of image-wise modulated light which is then detected by a photo-electric convertor so as to generate an electric signal representation of the irradiated object.

This electric signal representation can then be processed in a signal processor and / or stored. It can further be applied to a display device or to a printer to generate a visible image on which an evaluation, e.g. a diagnosis in case of a medical image, can be performed.

Increasingly, radiography flat panel detectors (FPD) for so-called direct radiography (DR) are being used which can convert X-rays in a direct way (direct conversion direct radiography), or in an indirect way (indirect conversion direct radiography) into an electric signal representation.

In direct radiography, the radiography detectors convert X-rays directly into electric charges directly interacting with a photoconductive layer such as amorphous selenium (a-Se). In indirect conversion direct radiography, the radiography flat panel detectors have a scintillator layer consisting of CsI:TI or *Gd*₂*O*₂*S* which converts X-rays into light which then interacts with the sensor being an amorphous silicon (a-Si) semiconductor layer, where electric charges are created.

It is evident that a patient or a body part to be examined (part of a patient such as a leg, an arm, ...) and the image recording apparatus have to be positioned relatively so that the radiation emitted by a source of radiation falls on the region of interest on the patient.

Commonly an operator guides the patient by oral commands to obtain the optimal position for image recording. For some exams, the operator guides the patient with his hands until the body part is in the correct position. Once the patient is positioned correctly for a certain examination type, the irradiation procedure is started. All these actions are time-consuming. Since these actions need interpretation by the patient, they may be error prone and may have to be repeated and corrected.

A common mistake for inexperienced radiographers is non-optimal positioning of the patient. The positioning task can often by decomposed in several steps, where each of the steps involves the movement of a particular body part. Sometimes these movements are subtle and not obvious for interpretation by the patient or radiographer if only a static pose or 2D image is presented.

Apart from the patient positioning it may be necessary for some types of examinations that a patient performs certain pre-examination actions, e.g. certain body movements prior to attaining the optimum position for image recording.

For example, to ideally position the patient for a frontal anterior-posterior view of the pelvis when the patient lies in a supine position, following steps help to bring the main axis of the pelvis parallel to the detector. Starting from a normal supine position, the patient bends his legs to bring his feet as close as possible to his pelvis while keeping his feet together, with the sole of the foot on the table. Lifting the pelvis from the table and lowering the pelvis back on the table before stretching the legs, brings the pelvis in the optimal position for a frontal anterior-posterior view.

Also in this case, the operator needs to guide the patient by means of a sequence of oral commands or assist him manually which prevents the operator to perform other tasks.

These commands are time-consuming and may be misinterpreted by the patient in which case they are to be repeated until the correct pre-recording movements are performed and the optimal image recording position is obtained.

US 2015/0003674 discloses a method of providing location information regarding a target object. Current location information and recommended location information are acquired and compared. Next additional information about the current location based on this comparison is outputted.

The location information of the target object is generated by means of a 2D camera or 2D stereo cameras or a depth camera.

The recommended location information is given by means of a reference image showing the correct position and pose for a certain examination type.

The result of the comparison is e.g. shown as an overlay image and a guide message (e.g. "please lift your arm").

When the comparison reveals that the patient is correctly positioned an emoticon such as a smiley may be displayed. If there is no exact overlap, the target gets an instruction how to reposition. Such instructions can be given e.g. by a message, a picture (e.g. an arrow indicating the direction in which the patient should move) or an animation image or a moving picture.

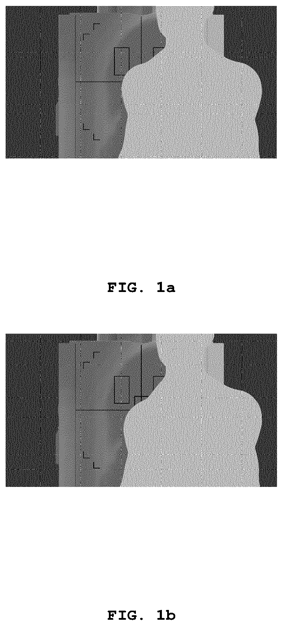

However, some of the instructions cannot be easily interpreted when showing only a 2D silhouette. For example, lowering the shoulder and arm which is needed for a correct rockwood projection is very difficult to illustrate by displaying only a 2D silhouette. As is illustrated in figures 1a and 1b, the transition from position A to position B could also be interpreted as rotating further into a lateral position rather than lowering the shoulder.

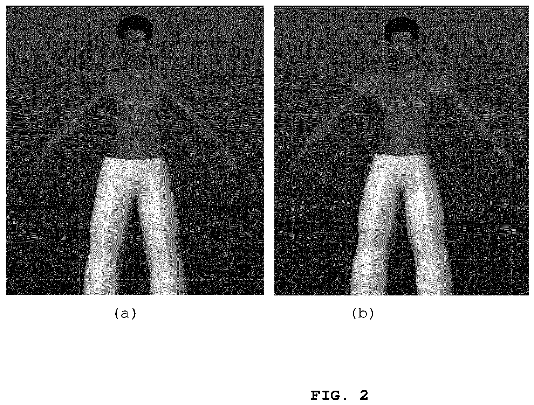

It can be easily seen that for the same rockwood position, the anatomy of the patient can make the instruction by 2D silhouettes ambiguous. For the body types displayed in the figures 2 (a) and (b), the silhouettes for a shoulder positioning is entirely different.

In patent US 2015/0003674 patient specific constraints are used which is also a drawback of the described method. Referring to the same rockwood positioning, the amount that a patient can lower its shoulder might be different depending on the mobility of the patient. An elderly patient, inhibited in his movement by his arthritis, could only lower his shoulder for a couple of cm, while a fit patient, trained in flexibility during his weekly yoga sessions, could possible lower his shoulder for ten cm, hereby opening the gap between head of the humerus and the glenoid as far as possible. Working with absolute positions would be a drawback in this situation.

It is an aspect of the present invention to overcome the above-described drawbacks.

The above-mentioned aspects are realized by a method having the specific method steps set out in claim 1. Specific features for preferred embodiments of the invention are set out in the dependent claims.

Further advantages and embodiments of the present invention will become apparent from the following description and drawings.

### Brief description of drawings

Fig. 1 a and b show 2 silhouettes were the silhouette shown in Fig.1 (b) is a silhouette of the patient shown in Fig. 1(a) but with his right shoulder lowered.
Fig. 2 shows two avatars created with MakeHuman (http://www.makehumancommunity.org/) where body type parameters are varied so as to have an effect on the appearance or shape of the shoulder.
Fig. 3-7 illustrates an example of a workflow according to the present invention,

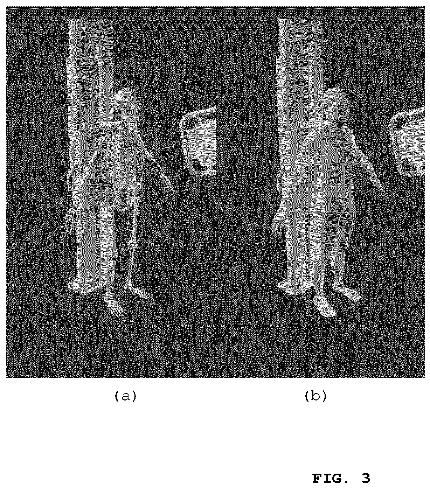

   Fig. 3: Stand against bucky

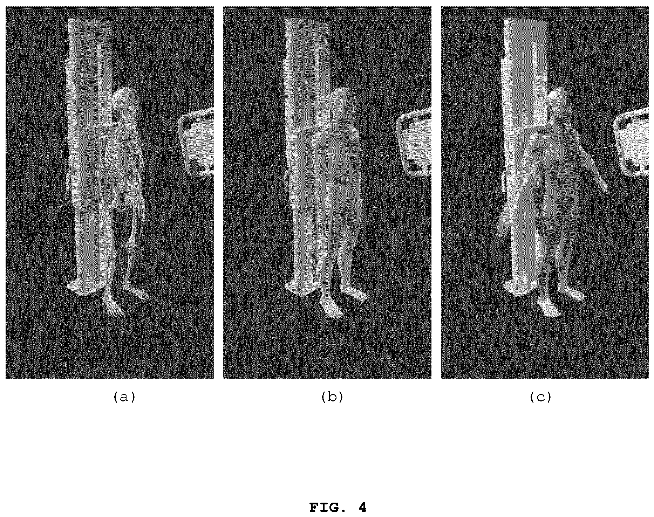

   Fig. 4: Lower your arms

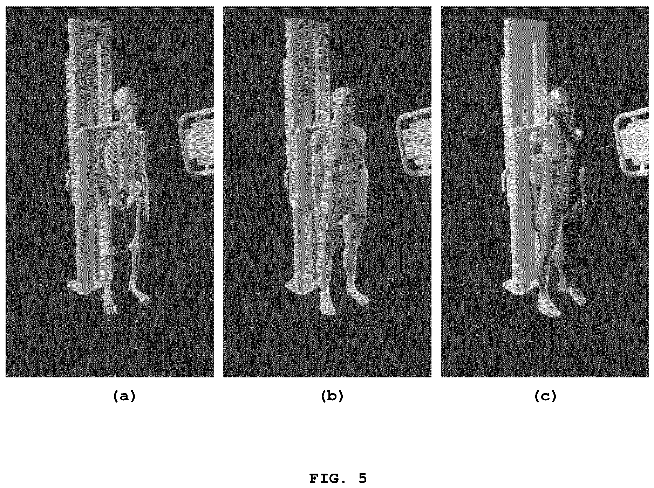

   Fig. 5: Bring your shoulder blade parallel to the detector

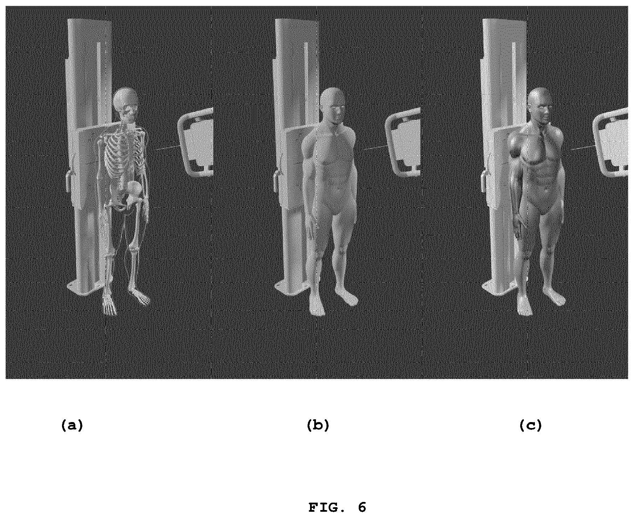

   Fig. 6: Lower your shoulder

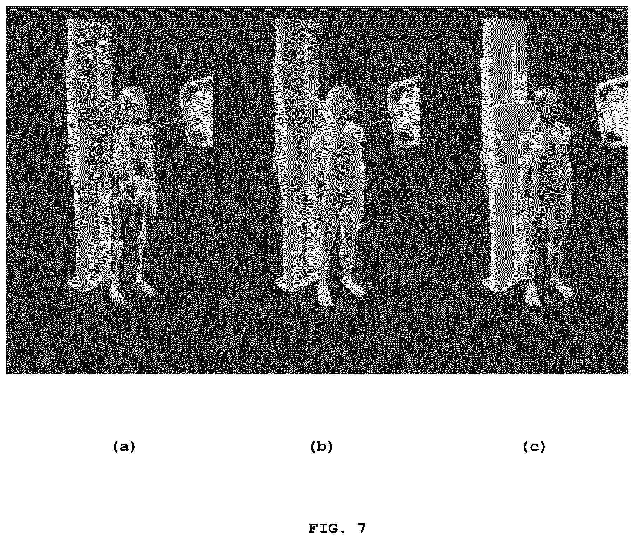

   Fig. 7: Look away from the X-ray source

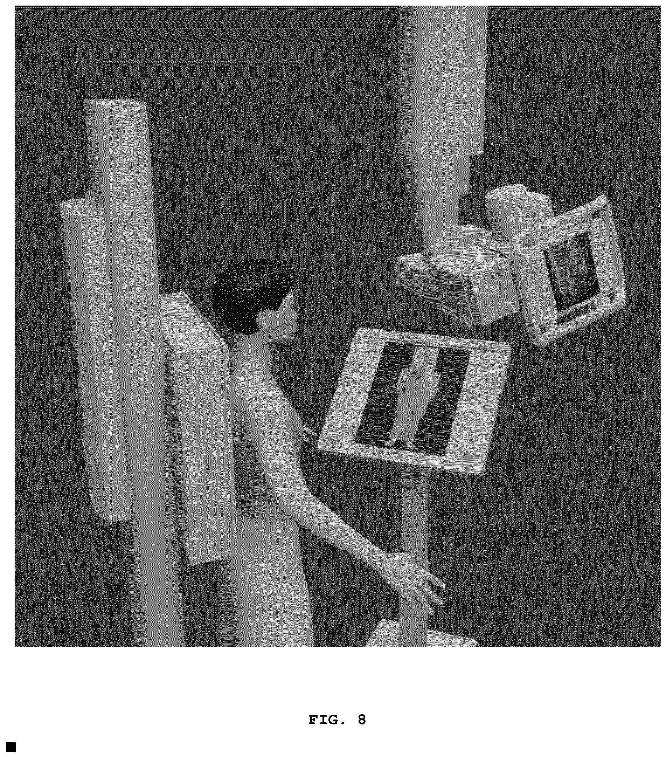

Fig. 8 shows an example of a modality with a monitor where the patient can see the avatar illustrating the necessary movements. On the tube head screen, the instructions for the radiographer are shown.

### Detailed description of the invention

The invention thus relates to the x-ray image recording of a patient or part of a patient, further on referred to as body part. The invention will be explained with reference to a human body. However, it will be clear that the invention is also applicable in the field of veterinary applications.

X-ray image recording is performed by means of an X-ray recording apparatus which generally consists of a source of radiation, a collimator provided in front of the source of radiation for confining the radiation emitted by the source of radiation to a specific field of interest, a user console coupled to the source of radiation for controlling the operation of the source and the collimator. Means are provided for supporting the x-ray source and means are provided for supporting the patient or the body part to be irradiated. These means are movable relative to each other.

It will be clear that in order to be able to generate an appropriate x-ray image of a patient or body part, the patient or the body part needs to be correctly positioned relative to the source of radiation so that irradiation of the intended body part is obtained.

To obtain correct positioning the first step of this invention is determining the current pose and location of a patient or of a part of a patient being positioned on a support of an x-ray image recording apparatus.

In the context of the present invention the term "location" refers to the place where the patient or the body part to be irradiated is located (positioned). By the term 'pose' is meant the way in which he stands in that position or in which the body part to be irradiated stands / lies in that position.

To this end a 3D image of the patient is recorded. A 3D camera, a depth camera or a combination of 2D cameras can be used to generate this image. By analysis of the images, the position of the patient or body part(s) can be determined. The pose of the patient can be reconstructed from these body part positions.

Irrespective of what type of image is used, it is important for the method of the present invention is that the pose and location of the patient or of the body part to be examined can be deduced from the recorded image. Deducing pose and locations constitutes the next step of the invention.

Different means can be used to determine pose and location. For example, 3D human pose estimation methods based on deep learning can be used. Such methods are described in e.g Jinbao Wang, Shujie Tan, Xiantong Zhen, Shuo Xu, Feng Zheng, Zhenyu He, Ling Shao, Deep 3D human pose estimation: A review, Computer Vision and Image Understanding, Volume 210, 2021, 103225, ISSN 1077-3142, https://doi.org/10.1016/j.cviu.2021.103225.

Pose and location of a patient or a body part can for example also be obtained by identifying the position of bones and bone joints in the recorded image or part thereof. Position of bones and bone joints can be obtained for example by subjecting the recorded image to a computer-implemented 2D pose estimation process.

The "OpenPose" algorithm is an example of such a pose estimation method. This algorithm has been described by Z. Cao, G. Hidalgo Martinez, T. Simon, S. Wei and Y.A. Sheikh in IEEE Transactions on Pattern Analysis and Machine Intelligence, OpenPose: Realtime Multi-Person 2D Pose Estimation using Part Affinity Fields, 2019.

If the location of the keypoints is known in the 2D image, the exact position of this joint or bodypart can be retrieved from a registered 3D image recorded with the 3D camera.

Other methods are also possible. The algorithm named "DensePose" segments the image and maps a 3D model to the segmentation parts in the image.

The "DensePose" algorithm has been described in the article "Densepose: Dense Human Pose Estimation in The Wild " by Riza Alp Guler, Natalia Neverova and lasonas Kokkinos in IEEE Conference on Computer Vision and Pattern Recognition, 2018.

Depending on the exam type, it is possible to use a method which is most accurate for that specific exam type.

Next for a specific examination type or for a number of examination types a description of the required position and pose for X-ray image recording of a patient or body part is set out (and stored) in advance.

This description may consist of the optimal pose and location for the patient or body part but it can additionally comprise margins within which the current pose and location may deviate from the optimal pose and location for a certain examination. For example, the adequate bending of an elbow for a certain examination may be specified as a range of bending angles which are acceptable for this type of examination.

In the following step the actual location and pose of a patient or a body part is compared with a description of pose and location for X-ray image recording of the specific examination type that is performed, which may be either the exact intended position and pose or with data regarding a range of allowable margins for said position and pose,

Then an instruction movie (video) is generated which displays at least movement and pose changes of the patient or the body part which are necessary for evolving from the current recorded position and pose towards the position and pose set out in said description or towards a position and pose which falls within the margins that are set out in the above described description.

The instruction video may thus guide the patient (or at least the body part of the patient) towards the optimal position and pose or guide the patient (or at least a body part of the patient) towards a position and pose which falls within the margins described in the above-mentioned description.

This instruction video preferably shows an Avatar of the patient or of the body part which moves from the current, detected pose and location of the patient towards the pose and location described as adequate for X-ray image recording (as described higher).

An avatar is 3D human model representing the patient within the setting of the room. Different avatars can be used. For example, the avatar being displayed to the patient most likely is a realistic rendering of a human model. The avatar displayed on the monitor facing the radiographer, might display the skeletal bones within a transparent version of the human model presented to the user.

3D computer graphics software which are suitable for generating such a video are commercially available. Examples are e.g. Maya (https://www.autodesk.com/products/mava/overview), Blender (https://www.blender.org/), Cinema 4D (https://www.maxon.net/en/cinema-4d), Houdini (https://www.sidefx.com/products/houdini/), ...

In one embodiment an Avatar in a displayed video thus shows the necessary movements of the patient or body part to be irradiated and to be imaged to reach the optimal position or a position which falls within the margins set out in the above-mentioned description.

For example, the necessary movements to achieve the optimal position for a rockwood position when started from a standing rest position are
a. Press your upper body against the bucky
b. Lower your arms to your body
c. Rotate your body until your shoulder blade is parallel to the bucky
d. Lower the arm or shoulder being imaged as much as possible
e. Look away from the X-ray source

The images of figure 3-7 show successive positions and / or poses the patient (or body part to be examined) has to take, which are the steps described above. The first picture (figures (a)) in this series is an avatar rendered with a skeleton and second and third pictures (figures (b) and (c)) are an avatar rendered with skin. This embodiment is not limitative for the present invention.

If a third picture in figure 3-7 is present, it shows the starting position of the avatar rendered with a transparent skin and the target position of the particular step. When looking at these rendering it becomes clear that the subtlety of steps c, d and e are not easily captured with still images. The same is true if only silhouettes, either still or moving, are used to illustrate these steps.

When assembling the steps in a video sequence, it becomes very clear and intuitive on which step needs to be performed. This makes from the video technique a very useful tool to illustrate to the patient or to the operator the successive poses and positions that lead towards an adequate pose and location for radiation image recording.

In between steps d and e of the workflow described higher, the radiographer will have to position the DR panel correctly. E.g. the bucky on which the patient is positioned has to be aligned in such a way that center of the DR panel is positioned between the head of the humerus and glenoid. The X-ray tube needs to inclined by about 17 degrees.

The steps which are needed to be performed by the radiographer can also be illustrated by an animation of the modality (X-ray image recording assembly). This is similar to the above-described animations of the avatar.

It's also possible to add an avatar of the radiographer which performs all the necessary tasks needed by the radiographer.

However, it is also possible that the avatar only shows the movement of that part of the patient or of the body part that needs to change pose and location to fall within the margins described in the above-mentioned description.

For example: after the execution of steps a to d of the workflow described higher and after the correct alignment of the bucky, the only step that the patient has to perform is the looking away from the detector. If the patient stays positioned correctly with respect to the position of his shoulder, the avatar will be animated and the patient sees a movie of the avatar which turns his head away from the X-ray source.

The instruction video showing the moving avatar can be displayed for viewing by the operator of the X-ray modality used for recording the X-ray image of the patient or body part.

However, since the avatar movement is self-explanatory; it is preferably directly shown to the patient so that he can imitate the movements of the avatar which are shown in the instruction video without any intervention of the operator. This mode of operation is direct, easy, more efficient and avoids mistakes.

In a preferred embodiment the instruction video also shows additional pre-examination movements to be performed by a patient or by a body part prior to the x-ray image recording. In another embodiment the avatar also shows additional predetermined recording requirements (e.g. hold breath) for certain examination types.

The above-mentioned pre-examination movements have as a goal to align the bones or open small gaps between the bones being examined.

For example; to align the pelvic bone parallel to the table, pre-examination steps mentioned above are necessary.

Finally, when the patient's pose and location matches that pre-scribed in the above-described description, an indication can be shown to the patient that no further adjustments of pose and location are required.

Next emission of radiation by the source of radiation can be initiated and the radiation image can be taken.

## Claims

1. Method of guiding a patient to a position suitable for x-ray image recording comprising the steps of
- Real time recording a 3D image of a patient in a position for X-ray image recording,
- Deriving from said 3D image the current position and pose of said patient,
- Comparing said current position and pose with a description of a range of allowable margins for position and pose for X-ray image recording,
- Generating an instruction video in which at least position and pose changes for said patient or part thereof which are necessary for moving from the current position and pose towards the position and pose set out in said description, are visualized.

2. Method according to claim 1 wherein said position and pose changes are visualized by means of position and pose changes of an avatar of at least part of said patient starting from an initial position and pose corresponding with the current position and pose of the patient and moving towards a position and pose which falls within said description.

3. Method according to claim 1 wherein said instruction video is displayed for viewing by said patient.

4. Method according to claim 1 wherein said instruction video is displayed for viewing by an operator of an X-ray modality used for recording said X-ray image.

5. Method according to claim 2 wherein said instruction video shows predetermined pre-recording movements for certain examination types by having said avatar additionally perform said pre-recording movements.

6. Method according to claim 2 wherein said instruction video shows predetermined recording requirements for certain examination types by having said avatar additionally perform said recording requirements.
